# EUROPEAN PATENT SPECIFICATION

(11) **EP 4 387 699 B1**
(45) Date of publication and mention of the grant of the patent: **10.06.2026**
(21) Application number: 21954404.6
(22) Date of filing: 20.08.2021
(51) Int. Cl.: A61M 27/00, H01H 71/00, A61F 9/007

(54) **ADJUSTABLE SHUNTING SYSTEMS WITH ENERGY REDIRECTING ELEMENTS**
EINSTELLBARE RANGIERSYSTEME MIT ENERGIEUMLEITENDEN ELEMENTEN
SYSTÈMES DE DÉRIVATION RÉGLABLES PRÉSENTANT DES ÉLÉMENTS DE REDIRECTION D'ÉNERGIE

(43) Date of publication of application: 26.06.2024
(73) Proprietor: Shifamed Holdings, LLC, Campbell, CA 95008 (US)
(72) Inventor: SAUL, Tom, Campbell, California 95008 (US)
(74) Representative: Mathys & Squire
(86) International application number: PCT/US2021/047013
(87) International publication number: WO 2023/022737

(56) References cited:
- WO-A1-2021/072317
- US-A1- 2006 173 399
- US-A1- 2011 152 752
- US-A1- 2018 028 794
- US-A1- 2018 028 794
- US-A1- 2021 116 778
- US-A1- 2021 251 806
- US-A1- 2021 251 806

## Description

### TECHNICAL FIELD

The present technology generally relates to implantable medical devices and, in particular, to adjustable shunting systems and associated methods for selectively controlling fluid flow between a first body region and a second body region of a patient.

### BACKGROUND

Implantable shunting systems are widely used to treat a variety of patient conditions by shunting fluid from a first body region/cavity to a second body region/cavity. The flow of fluid through the shunting systems is primarily controlled by the pressure gradient across the shunt and the physical characteristics of the flow path defined through the shunt (e.g., the resistance of the shunt lumen(s)). However, most shunting systems have a single static flow path that is not adjustable. Accordingly, one challenge with conventional shunting systems is selecting the appropriate size shunt for a particular patient. A shunt that is too small may not provide enough therapy to the patient, while a shunt that is too large may create new issues in the patient. Despite this, most conventional shunts cannot be adjusted after implantation and, therefore, cannot be adjusted or titrated to meet the patient's individual and variable needs and/or to account for changes in flow-related characteristics, such as flow volume, inflow pressure, and/or outflow resistance.

WO 2021/ 072317 describes adjustable flow glaucoma shunts. The shunts include a drainage element configured to fluidly couple an anterior chamber of an eye and a target outflow location, such as a subconjunctival bleb space. The shunts further include a flow control assembly coupled to the drainage element and configured to control the flow of fluid (e.g., aqueous) therethrough. The shunts further include an outer membrane or bladder that encases the flow control assembly. The outer membrane includes a plurality of apertures that fluidly couple an interior of the outer membrane with an environment exterior to the outer membrane.

US 2021/251806 describes intraocular shunting systems that include a drainage element having an inflow portion configured for placement within an anterior chamber of the eye outside of an optical field of view of the patient and an outflow portion configured for placement at a different location of the eye. The system includes a flow control assembly having a rotational control element operably coupled to the drainage element. The flow control assembly includes an actuation structure coupled to the rotational control element and configured to selectively change an orientation of the rotational control element.

The present invention is set out in the appended claims.

### BRIEF DESCRIPTION OF THE DRAWINGS

Many aspects of the present technology can be better understood with reference to the following drawings. The components in the drawings are not necessarily drawn to scale. Instead, emphasis is placed on illustrating clearly the principles of the present technology. Furthermore, components can be shown as transparent in certain views for clarity of illustration only and not to indicate that the component is necessarily transparent. Components may also be shown schematically.
FIG. 1A illustrates an adjustable shunting system configured in accordance with select embodiments of the present technology.
FIG. 1B is an enlarged view of a plate assembly of the system of FIG. 1A and configured in accordance with select embodiments of the present technology.
FIG. 2A illustrates an adjustable shunting system including redirecting elements in accordance with select embodiments of the present technology.
FIG. 2B illustrates a cross-sectional view of the adjustable shunting system of FIG. 2A taken along section line 2B-2B.
FIG. 2C is an enlarged view of region 2C of the adjustable shunting system of FIG. 2A.
FIG. 2D is a perspective view of the plate assembly and redirecting elements of FIG. 2C with other aspects of the adjustable shunting system omitted for clarity.
FIG. 2E is a cross-sectional view of the plate assembly and redirecting elements of FIG. 2D taken along section line 2E-2E.

### DETAILED DESCRIPTION

The present technology is generally directed to adjustable shunting systems, including adjustable shunting systems having energy redirecting elements ("the redirecting elements"). The redirecting elements can be configured to redirect, reflect, refract, convey, or otherwise transmit energy (e.g., optical, laser, or other energy) received from an energy source spaced apart from the shunting system toward a target region of the shunting system. For example, the shunting system can include one or more actuators for controlling the flow of fluid through the system, and the redirecting elements can direct energy to the one or more actuators. In particular, an end region of the redirecting element can be at least partially aligned with a shape-memory actuation element of the actuator such that energy applied to the redirecting element is transmitted to the shape-memory actuation element to drive actuation thereof.

As described in greater detail below, it is expected that in at least some embodiments the present technology may exhibit one or more advantageous characteristics that improve operation of shape-memory actuators and/or adjustable shunting systems. For example, conventional adjustable shunting systems may require direct access (e.g., via line of sight) to the associated actuation elements to apply the energy to drive actuation thereof. In contrast with conventional systems, redirecting elements configured in accordance with embodiments of the present technology can transmit energy to the shape-memory actuation elements, such that the actuation elements are expected to be accessible to energy applied from a secondary location (e.g., external to the patient) even if the actuation element is generally not directly accessible (e.g., not within line of sight) from the secondary location. Additionally, the redirecting elements described herein can be positioned at least partially in a different body cavity/region than the actuators. In some embodiments, the different body cavity/region can be easier/more readily accessible such that it is easier to deliver energy to the redirecting elements. In some embodiments, the different body cavity/region can be associated with a reduced likelihood of risk or damage to other (e.g., surrounding, adjacent, proximate, etc.) portions of a patient's anatomy during the energy delivery process. Furthermore, the redirecting elements can be configured to reduce (e.g., minimize) energy loss during transmission and/or interference with other elements of the adjustable shunting system and/or the patient's anatomy. Of course, the present technology may also provide additional advantageous characteristics not expressly described herein.

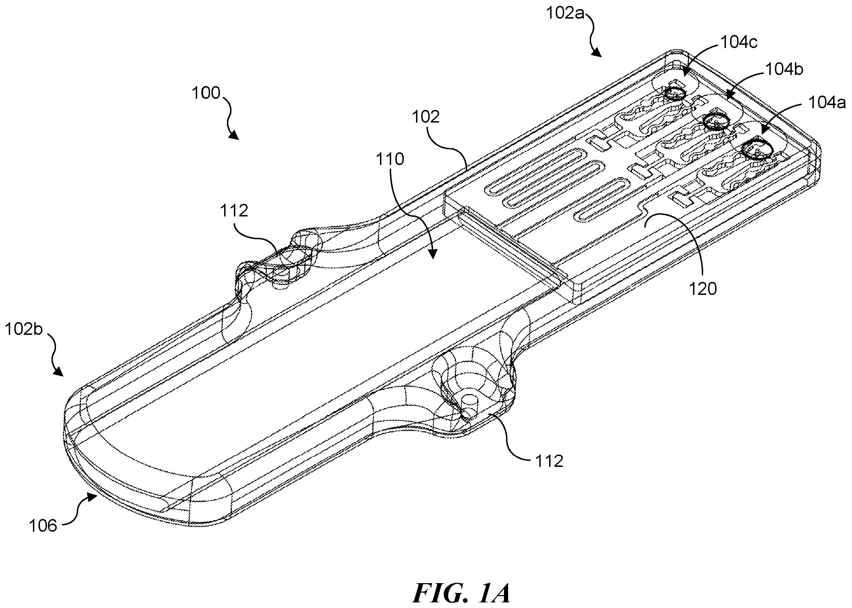

The terminology used in the description presented below is intended to be interpreted in its broadest reasonable manner, even though it is being used in conjunction with a detailed description of certain specific embodiments of the present technology. Certain terms may even be emphasized below; however, any terminology intended to be interpreted in any restricted manner will be overtly and specifically defined as such in this Detailed Description section. Additionally, the present technology can include other embodiments that are within the scope of the examples but are not described in detail with respect to FIGS. 1A-2E.

Reference throughout this specification to "one embodiment" or "an embodiment" means that a particular feature, structure, or characteristic described in connection with the embodiment is included in at least one embodiment of the present technology. Thus, the appearances of the phrases "in one embodiment" or "in an embodiment" in various places throughout this specification are not necessarily all referring to the same embodiment. Furthermore, the particular features or characteristics may be combined in any suitable manner in one or more embodiments.

Reference throughout this specification to relative terms such as, for example, "generally," "approximately," and "about" are used herein to mean the stated value plus or minus 10%. Reference throughout this specification to the term "resistance" refers to fluid resistance unless the context clearly dictates otherwise. The terms "drainage rate" and "flow rate" are used interchangeably to describe the movement of fluid through a structure at a particular volumetric rate. The term "flow" is used herein to refer to the motion of fluid, in general.

Although certain embodiments herein are described in terms of shunting fluid from an anterior chamber of an eye, one of skill in the art will appreciate that the present technology can be readily adapted to shunt fluid from and/or between other portions of the eye, or, more generally, from and/or between a first body region and a second body region. Moreover, while the certain embodiments herein are described in the context of glaucoma treatment, any of the embodiments herein, including those referred to as "glaucoma shunts" or "glaucoma devices" may nevertheless be used and/or modified to treat other diseases or conditions, including other diseases or conditions of the eye or other body regions. For example, the systems described herein can be used to treat diseases characterized by increased pressure and/or fluid build-up, including but not limited to heart failure (e.g., heart failure with preserved ejection fraction, heart failure with reduced ejection fraction, etc.), pulmonary failure, renal failure, hydrocephalus, and the like. Moreover, while generally described in terms of shunting aqueous, the systems described herein may be applied equally to shunting other fluid, such as blood or cerebrospinal fluid, between the first body region and the second body region.

FIG. 1A illustrates a shunting system 100 ("the system 100") configured in accordance with select embodiments of the present technology. As described in greater detail below, the system 100 is configured to provide an adjustable therapy for draining fluid from a first body region to a second, different body region, such as to drain aqueous from an anterior chamber of a patient's eye.

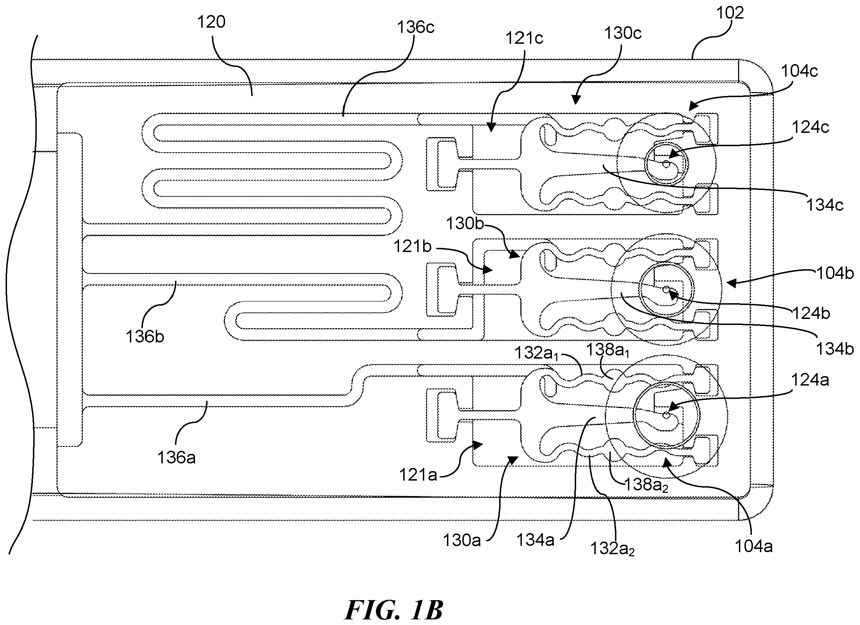

The system 100 includes a generally elongated housing 102 and a plate assembly 120. The elongated housing 102 (which can also be referred to as a casing, membrane, shunting element, or the like) extends between a first end portion 102a and a second end portion 102b. The elongated housing 102 substantially and/or fully encases the plate assembly 120 at or proximate the first end portion 102a. In the illustrated embodiment, the elongated housing 102 includes three openings 104 (e.g., a first opening 104a, a second opening 104b, and a third opening 104c) that align with respective fluid inlets in the plate assembly 120, as described in further detail below with respect to FIG. 1B. The elongated housing 102 further includes a main fluid conduit 110 fluidly coupling the plate assembly 120 to one or more fluid outlets 106 positioned proximate the second end portion 102b of the elongated housing 102. In some embodiments, the elongated housing 102 is composed of a slightly elastic or flexible biocompatible material (e.g., silicone, etc.). The elongated housing 102 can also optionally have one or more wings or appendages 112 having holes (e.g., suture holes) for securing the elongated housing 102 in a desired position.

The plate assembly 120 (which can also be referred to as a flow control plate, a flow control cartridge, a plate structure, or the like) is positioned within the elongated housing 102 and is configured to control the flow of fluid through the system 100. For example, as best seen in FIG. 1B, the plate assembly 120 includes one or more fluid apertures or inlets 124 (e.g., a first fluid inlet 124a, a second fluid inlet 124b, and a third fluid inlet 124c) that align with corresponding openings 104a-c in the elongated housing 102. The fluid inlets 124 permit fluid to enter an interior of the plate assembly 120 (and thus an interior of the elongated housing 102) from an environment external to the system 100. In some embodiments, an upper surface of the plate assembly 120 forms a substantial fluid seal with an interior surface of the elongated housing 102 at the first end portion 102a such that the only way for fluid to enter the system 100 is through the fluid inlets 124. Accordingly, for fluid to flow through the system 100, it generally must flow through the plate assembly 120.

The fluid path through the plate assembly 120 depends on which fluid inlet 124 the fluid enters through. For example, fluid that enters the system 100 via the first fluid inlet 124a flows into a first chamber 121a of the plate assembly 120 and drains to the main fluid conduit 110 via a first channel 136a. Fluid that enters the system 100 via the second fluid inlet 124b flows into a second chamber 121b of the plate assembly 120 and drains to the main fluid conduit 110 via a second channel 136b. Fluid that enters the system 100 via the third fluid inlet 124c flows into a third chamber 121c of the plate assembly and drains to the main fluid conduit 110 via a third channel 136c. The chambers 121a-c can be fluidly isolated such that there are three discrete flow paths through the plate assembly 120. The channels 136a-c can also have different geometric configurations (e.g., lengths) relative to one another such that they have different fluid resistances, and thus provide different flow rates for a given pressure. The relative level of therapy provided by each fluid path can be different so that a user may adjust the level of therapy provided by the system 100 by selectively opening and/or closing various fluid paths (e.g., by selectively interfering with or permitting flow through individual fluid inlets 124), as described below. For example, under a given pressure, when fluid enters primarily through the first fluid inlet 124a, the system 100 can provide a first drainage rate, when fluid enters primarily through the second fluid inlet 124b, the system 100 can provide a second drainage rate less than the first drainage rate, and when fluid primarily enters through the third fluid inlet 124c, the system 100 can provide a third drainage rate less than the first drainage rate. In other embodiments, the channels 136a-c can have the same or generally the same geometric configurations such that they have the same or generally the same fluid resistances, and thus provide similar flow rates for a given pressure.

The plate assembly 120 is configured to selectively control the flow of fluid entering the system 100. In particular, the plate assembly 120 includes a first actuator 130a positioned in the first chamber 121a and configured to control the flow of fluid through the first fluid inlet 124a, a second actuator 130b positioned in the second chamber 121b and configured to control the flow of fluid through the second fluid inlet 124b, and a third actuator 130c positioned in the third chamber 121c and configured to control the flow of fluid through the third fluid inlet 124c. The first actuator 130a can include a first projection or gating element 134a configured to moveably interface with the first fluid inlet 124a, e.g., to move between a first (e.g., "open") position in which the gating element 134a does not substantially prevent fluid from flowing through the first fluid inlet 124a (e.g., by not interfering with the first fluid inlet 124a) and a second (e.g., "closed") position in which the gating element 134a substantially prevents fluid from flowing through the first fluid inlet 124a (e.g., by blocking the first fluid inlet 124a). In some embodiments, the gating element 134a can be configured to move to one or more intermediate positions between the first (e.g., open) and the second (e.g., closed) position. The second actuator 130b can include a second gating element 134b and the third actuator 130c can include a third gating element 134c that operate in a similar manner as the gating element 134a (e.g., moveable between open and closed positions relative to the second fluid inlet 124b and the third fluid inlet 124c).

The first actuator 130a can further include a first actuation element 132a₁ and a second actuation element 132a₂ that drive movement of the gating element 134a between the first (e.g., open) position and the second (e.g., closed) position. The first actuation element 132a₁ and the second actuation element 132a₂ can be composed at least partially of a shape memory material or alloy (e.g., nitinol). Accordingly, the first actuation element 132a₁ and the second actuation element 132a₂ can be transitionable at least between a first material phase or state (e.g., a martensitic state, a R-phase, a composite state between martensitic and R-phase, etc.) and a second material phase or state (e.g., an austenitic state, an R-phase state, a composite state between austenitic and R-phase, etc.). In the first material state, the first actuation element 132a₁ and the second actuation element 132a₂ may have reduced (e.g., relatively less stiff) mechanical properties that cause the actuation elements to be more easily deformable (e.g., compressible, expandable, etc.) relative to when the actuation elements are in the first material state. In the second material state, the first actuation element 132a₁ and the second actuation element 132a₂ may have increased (e.g., relatively stiffer) mechanical properties relative to the first material state, causing an increased preference toward a specific preferred geometry (e.g., original geometry, manufactured or fabricated geometry, heat set geometry, etc.). The first actuation element 132a₁ and the second actuation element 132a₂ can be selectively and independently transitioned between the first material state and the second material state by applying energy (e.g., laser energy, electrical energy, etc.) to the first actuation element 132a₁ or the second actuation element 132a₂ to heat it above a transition temperature (e.g., above an austenite finish (A_{f}) temperature, which is generally greater than body temperature). If the first actuation element 132a₁ (or the second actuation element 132a₂) is deformed relative to its preferred geometry when heated above the transition temperature, the first actuation element 132a₁ (or the second actuation element 132a₂) will move to and/or toward its preferred geometry. In some embodiments, the first actuation element 132a₁ and the second actuation element 132a₂ are operably coupled such that, when the actuated actuation element (e.g., the first actuation element 132a₁) transitions toward its preferred geometry, the non-actuated actuation element (e.g., the second actuation element 132a₂) is further deformed relative to its preferred geometry.

The first actuation element 132a₁ and the second actuation element 132a₂ generally act in opposition. For example, the first actuation element 132a₁ can be actuated to move the gating element 134a to and/or toward the first (e.g., open) position, and the second actuation element 132a₂ can be actuated to move the gating element 134a to and/or toward the second (e.g., closed) position. Additionally, as described above, the first actuation element 132a₁ and the second actuation element 132a₂ can be coupled such that as one moves toward its preferred geometry upon material phase transition, the other is deformed relative to its preferred geometry. This enables the actuation elements 132a to be repeatedly actuated and the gating element 134a to be repeatedly cycled between the first (e.g., open) position and the second (e.g., closed) position.

In some embodiments, each actuation element 132 can include one or more targets 138. The target(s) 138 can be thermally coupled to the corresponding actuation element(s) 132 such that that energy (e.g., laser energy) received at the target(s) 138 can dissipate through the corresponding actuation element(s) 132 as heat. The target(s) 138 can therefore be selectively targeted with energy to actuate the actuation elements 132. For example, to actuate the first actuation element 132ai, heat/energy can be applied to the first target 138a₁, such as from an energy source positioned external to the patient's eye (e.g., a laser). The heat applied to the first target 138ai spreads through at least a portion of the first actuation element 132a₁, which can heat the first actuation element 132a₁ above its transition temperature. To actuate the second actuation element 132a₂ heat/energy can be applied to the second target 138a₂. The heat applied to the second target 138a₂ spreads through the second actuation element 132a₂, which can heat at least the portion of the second actuation element 132a₂ above its transition temperature. In the illustrated embodiment, the targets 138 are positioned generally centrally along a length of each individual actuation element 132. In other embodiments, the targets 138 can be positioned at an end region of each individual actuation element 132. In some embodiments, the targets 138 are composed of a same material (e.g., nitinol) as the actuation elements 132. Without being bound by theory, the increased surface area of the targets 138 relative to the actuation elements 132 is expected in increase the ease and consistency by which the actuators 130 can be actuated using an energy source (e.g., a laser) positioned external to the body.

The second actuator 130b and the third actuator 130c can also each include a pair of opposing shape-memory actuators and operate in the same or similar fashion as the first actuator 130a. Additional details regarding the operation of shape memory actuators, as well as adjustable glaucoma shunts, are described in U.S. Patent Application No. 17/175,332, U.S. Patent App. Publication No. 2020/0229982, and International Patent Application Nos. PCT/US20/55144, PCT/US20/55141, PCT/US21/14774, PCT/US21/18601, PCT/US21/23238, and PCT/US21/27742.

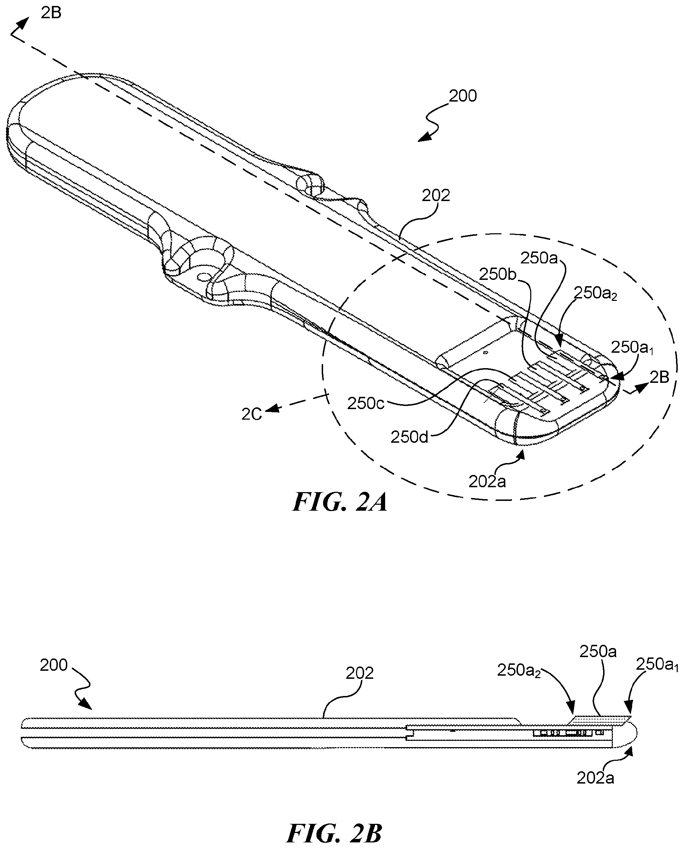

FIGS. 2A-2E illustrate an adjustable shunting system 200 ("the system 200") including energy redirecting elements 250a-d (referred to collectively as "the redirecting elements 250") in accordance with select embodiments of the present technology. The system 200 can include elements that are generally similar to or the same as elements of the system 100 of FIGS. 1A and 1B. Accordingly, like numbers are used to designate like elements (e.g., actuator 130 versus actuator 230), and the discussion of FIGS. 2A-2E will be limited to those features that differ from FIGS. 1A and 1B and any additional aspects necessary for context.

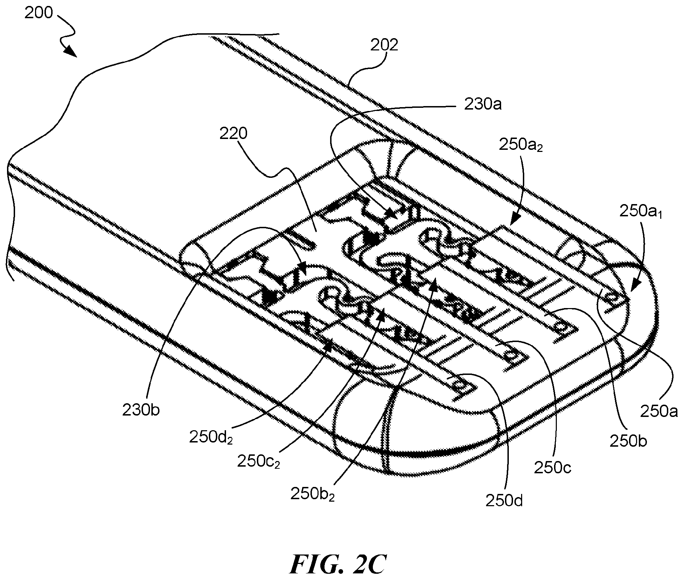

Referring first to FIG. 2A, the system 200 can include one or more redirecting elements 250. In the illustrated embodiment, for example, the system 200 includes a first redirecting element 250a, a second redirecting element 250b, a third redirecting element 250c, and a fourth redirecting element 250d. FIG. 2B illustrates a cross-sectional view of the adjustable shunting system 200 of FIG. 2A taken along section line 2B-2B. Referring to FIGS. 2A and 2B together, the redirecting elements 250 can be positioned near a first end portion 202a of the housing 202. Each of the redirecting elements 250 can have a first end portion and a second end portion. In the illustrated embodiment, for example, the first redirecting element 250a has a first end portion 250a₁ and a second end portion 250a₂ opposite the first end portion 250a₁. In the illustrated embodiment, the first end portion 250a₁ is positioned proximate the first end portion 202a of the housing 202. In other embodiments, the first end portion 250ai can have other suitable positions. For example, the first end portion 250ai can extend past the first end portion 202a of the housing such that at least a portion of the first redirecting element 250 does not overlap with the housing 202. In yet other embodiments, the first end portion 250ai can extend angularly or perpendicularly relative to a longitudinal axis of the housing 202 such that the first end portion 250ai does not overlap with the housing 202. As described in detail below with respect to FIGS. 2C-2E, the second end portion 250a₂ can be aligned (e.g., vertically, axially, etc.) with an actuator contained within the housing 202. The second redirecting element 250b, the third redirecting element 250c, and the fourth redirecting element 250d can also have end portions generally similar to or the same as the end portions 250a₁₋₂ of the first redirecting element 250a.

In some embodiments, each of the redirecting elements 250 can be at least partially contained by and/or coupled to the housing 202. In the illustrated embodiment, for example, the first end portions of the redirecting elements 250a-d (e.g., first end portion 250a₁, etc.) are at least partially contained by and/or coupled to the first end portion 202a of the housing 202. In other embodiments, as described above, the first end portions of the redirecting elements 250 (e.g., first end portion 250a₁, etc.) can extend from and/or beyond the housing 202. In at least some embodiments, for example, the first end portions (e.g., first end portion 250a₁, etc.) of the redirecting elements 250 can extend beyond the first end portion 202a of the housing 202 in a direction generally or substantially parallel to a longitudinal axis of the housing 202. Additionally, although the redirecting elements 250 in FIGS. 2A-2B are illustrated as being generally or substantially aligned with (e.g., parallel to) a longitudinal axis of the system 200, in other embodiments one or more of the redirecting elements 250 can be angled, or have any other suitable orientation, relative to the longitudinal axis of the system 200. Regardless of the orientation and position of the redirecting elements 250, the redirecting elements 250 can be coupled (directly or indirectly) to the housing 202 to maintain a positional relationship between the redirecting elements 250 and the housing 202.

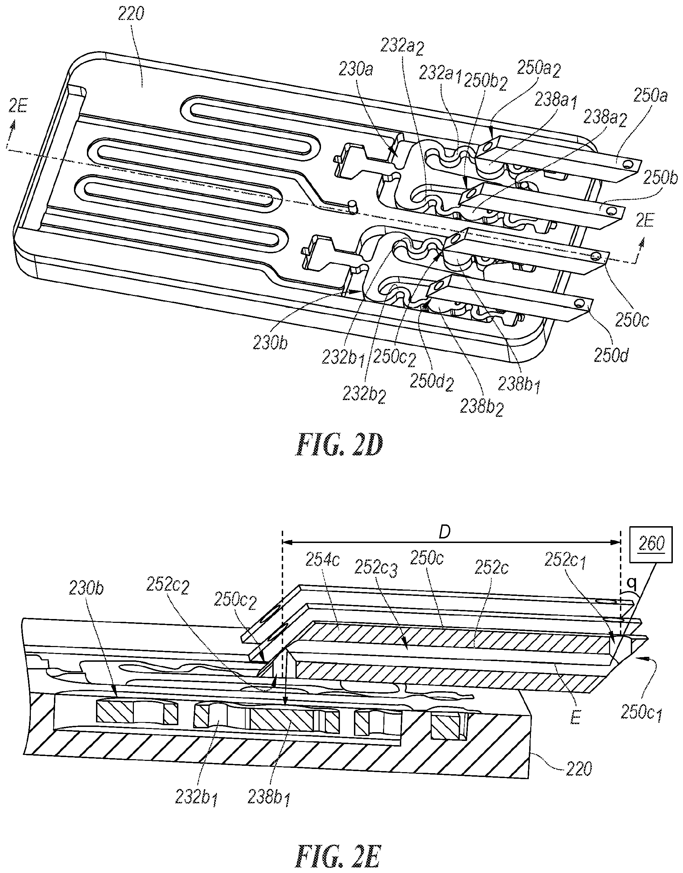

As described in greater detail below with reference to FIGS. 2D and 2E, each of the redirecting elements 250 can be configured to redirect, reflect, refract, convey, or otherwise transmit energy from the first end portion (e.g., the first end portion 250ai of the first redirecting element 250a, etc.) to the second end portion (e.g., the second end portion 250a₂ of the first redirecting element 250a, etc.). For example, the redirecting elements 250 can direct light or optical energy (e.g., laser energy) received at the first end portion to the second end portion. In some embodiments, the redirecting elements 250 can be configured to operate in reverse, e.g., to transmit energy from the second end portion to the first end portion.

FIG. 2C is an enlarged view of region 2C of the adjustable shunting system 200 of FIG. 2A. Similar to the system 100 described with respect to FIGS. 1A and 2B, the system 200 can include a plate assembly 220. In FIG. 2C, a portion of the housing 202 that covers the plate assembly 220 has been omitted for the purpose of clarity. The plate assembly 220 can include one or more actuators 230. In the illustrated embodiment, for example, the plate assembly 220 includes a first actuator 230a and a second actuator 230b. The second end portions of each of the redirecting elements 250 can correspond to and/or be at least partially aligned with (e.g., positioned above) one of the actuators 230. In the illustrated embodiment, for example, the first and second redirecting elements 250a, 250b correspond to the first actuator 230a such that the respective second end portions 250a₂, 250b₂ of the first and second redirecting elements 250a, 250b are aligned with the first actuator 230a. Similarly, in the illustrated embodiment, the third and fourth redirecting elements 250c, 250d correspond to the second actuator 230b such that the respective second end portions 250c₂, 250d₂ of the third and fourth redirecting elements 250c, 250d are aligned with the second actuator 230b. Accordingly, as described in greater detail below with reference to FIGS. 2D and 2E, each of the redirecting elements 250 can redirect energy to a corresponding one or the actuators 230, e.g., to actuate the corresponding actuator 230.

FIG. 2D is a perspective view of the plate assembly 220 and redirecting elements 250 with other aspects of the system 200 omitted for clarity. As best seen in FIG. 2D, each of the actuators 230 can include one or more actuation elements 232, and each of actuation elements 232 can include one or more targets 238. Additionally, each of the redirecting elements 250 can correspond to and/or be at least partially aligned with a corresponding one of the actuation elements 232 and/or a corresponding one of the targets 238. In the illustrated embodiment, for example, the first redirecting element 250a corresponds to a first actuation element 232a₁ of the first actuator 230a, such that the second end portion 250a₂ of the first redirecting element 250a is aligned with a first target 238a₁ of the first actuator 230a. Similarly, the second redirecting element 250b corresponds to a second actuation element 232a₂ of the first actuator 230a, such that the second end portion 250b₂ of the second redirecting element 250b is aligned with a second target 238a₂ of the first actuator 230a. The third redirecting element 250c and the fourth redirecting element 250d can be configured generally similar to or the same as the first redirecting element 250a and/or the second redirecting element 250b, but with the third and fourth redirecting elements 250c-d corresponding to respective first and second actuation elements 232b₁₋₂ and respective first and second targets 238b₁₋₂ of the second actuator 230b.

FIG. 2E is a cross-sectional view of the plate assembly 220 and third redirecting element 250c of FIG. 2D taken along section line 2E-2E. Although described in the context of the third redirecting element 250c, the following description of FIG. 2E applies equally to the first, second, and fourth redirecting elements 250a, 250b, 250d of FIGS. 2A-2D, and the corresponding actuators 230 and actuation elements 232.

In the embodiment illustrated in FIG. 2E, the third redirecting element 250c includes an interior or transmission component 252c at least partially contained, surrounded, or housed by an exterior component 254c. The transmission component 252c can include a first (e.g., input, target, receiving, etc.) region 252ci in the first end portion 250ci, a second (e.g., output) region 252c₂ in the second end portion 250c₂, and an intermediate region 252c₃ extending therebetween and coupling (e.g., communicatively, optically, etc.) the first and second regions 252c₁₋₂. As described above, the second region 252c₂ can be aligned with the corresponding actuation element 232 and/or the corresponding target 238, e.g., with the first target 238bi of the first actuation element 232b₁.

The redirecting elements 250 can be used to apply/transmit energy E to the corresponding actuation elements 232. As best seen in FIG. 2E, an energy source 260 can emit energy E to and/or toward the third redirecting element 250c. When the energy E is incident on the first region 252c₁, the energy can be redirected, reflected, refracted, conveyed, or otherwise transmitted from the first region 252ci to the second region 252c₂ via the intermediate region 252c₃. The energy E can exit the third redirecting element 250c via the second region 252c₂ such that the energy E is applied to the first target 238bi. The energy E received at the first target 238bi can heat the first actuation element 232bi, which, if heated above its transition temperature, may cause the first actuation element 232bi to move to and/or toward its preferred geometry, thereby moving a gating element (such as the gating elements 134a-b of FIG. 1B) between a first position and a second position, as described previously and with reference to FIGS. 1A and 1B. The redirecting elements 250 can therefore be used to selectively and independently actuate the actuation elements 232 via the targets 238 (e.g., by selectively applying energy E to individual ones of the redirecting elements 250 to transmit the energy E to the corresponding targets 238 and transition the corresponding actuation elements 232 from the first material state to the second material state), as described previously.

Without being bound by theory, the redirecting elements 250 are expected to improve the targeting of the actuation elements 232 with energy delivered from an energy source spaced apart from the system 200 (e.g., a laser positioned external to the patient). For example, because the redirecting elements 250 can transmit energy to the corresponding actuators, the first region 252ci of the redirecting element 250c can be positioned at least partially in a body cavity/region that is easier to deliver energy to and/or that is associated with a reduced likelihood of damage to other (e.g., surrounding, adjacent, proximate, etc.) portions of a patient's anatomy (e.g., eye, etc.) during the energy delivery process, while still allowing other components of the system 100, 200 (e.g., the actuators 230, the fluid inlets 104 of FIGS. 1A and 1B, the fluid outlet(s) 106 of FIGS. 1A, etc.) to be positioned at least partially in other body cavity(ies)/region(s) of a patient. Accordingly, the targets 238 are therefore expected to be accessible to energy E even if the body of one of the actuation elements 232 is generally not directly accessible. Additionally, in at least some embodiments, the redirecting elements 250 can at least partially insulate, isolate, shield or otherwise confine the energy E during transmission so as to reduce (e.g., minimize) energy loss during transmission, interference with other elements of the system 200, and/or interference with the patient's anatomy.

As a non-limiting example, the system 200 can be an adjustable glaucoma shunt with the first end portion 202a of the housing 202 (or at least a fluid inlet to the system 200) positioned in an anterior chamber of a patient's eye, and the second end portion 202b of the housing positioned in a desired outflow location (e.g., a bleb space). The energy redirecting elements 250 can extend into the anterior chamber a distance sufficient such that the first end portions of the redirecting elements 250 are accessible (e.g., via line of sight) and can therefore be targeted using a laser. However, some or all of the actuators 230 can be positioned at least partially outside the anterior chamber and can be inaccessible (e.g., via line of sight) and therefore cannot be targeted using a laser. Nonetheless, the actuators 230 can be actuated by using the energy redirecting elements 250, as described previously. Accordingly, the energy redirecting elements 250 can reduce the portion of the housing 202 that must be positioned within the anterior chamber and/or otherwise be visible in order for the system 200 to operate.

In some embodiments, the third redirecting element 250c can comprise an optical fiber or light tube. The optical fiber can be formed from one or more elements and/or materials. In at least some embodiments, for example, the transmission component 252c can be formed from a first material having a relatively high first refractive index (e.g., between about 1.3 and about 4, such as at least 1.34, 1.6 or any other suitable refractive index), such as high refractive index optical epoxy, polydimethylsiloxane (PDMS), BK7 glass, or any other suitable material. The exterior component 254c surrounding the transmission component 252c can be a second material having a relatively low second refractive index (e.g., less than the first refractive index, between about 1 and about 4, such as up to 1.34, 1.6, or any other suitable refractive index), such as glass or a low refractive silicone elastomer. In such embodiments, the energy E can be applied at an incidence angle q ("the angle q") from perpendicular relative to the first region 252ci without or substantially without reducing energy E transmission by the third redirecting element 250c, which can allow for a greater margin of error when aligning the energy E with the first region 252ci. The angle q can be between about 0 degrees and about 70 degrees, such as an angle of at least 1 degree, 5 degrees, 10 degrees, 15 degrees, 20 degrees, 25 degrees, 50 degrees, 60 degrees, 65 degrees, or any other suitable angle. In at least some embodiments, the angle q can be between about 50 degrees and about 70 degrees, such as between about 53 degrees and about 66 degrees. In some embodiments, the angle q can correspond to a refractive index of the transmission component 252c, such that the angle q (e.g., a numerical aperture of the first region 252ci) increases as the refractive index of the transmission component 252c increases.

In at least some embodiments, for example, the transmission component 252c can include one or more prisms, mirrors, and/or reflective surfaces positioned in the interior of the third redirecting element 250c. In such embodiments, the one or more prisms, mirrors, and/or reflective surfaces can be separated by a vacuum, air, water, and/or any other suitable fluid and/or material that permits energy (e.g., optical energy) to be propagated therethrough. In some embodiments, the third redirecting element 250c can be a prism, and the first, second, and intermediate regions 252c₁₋₃ can be portions or regions of the prism. In such embodiments, the energy E may need to be applied at an incidence angle q of about zero (e.g., generally or substantially perpendicular to the first region 252c₁), or any other suitable incidence angle q or range of incidence angles (e.g., based at least in part on the configuration of the prism), for the third redirecting element 250c to redirect/transmit energy E sufficient to actuate the second actuator 230b. In some embodiments, the third redirecting element 250c can include a plurality of mirrors and/or prisms coupled to the plate assembly 220 and/or the system 200 (FIGS. 2A-2C). In such embodiments, the first, second, and/or intermediate regions 252c₁₋₃ can each include one or more individual mirrors and/or prisms configured to redirect/transmit the energy E from the first region 252ci to the second region 252c₂.

The third redirecting element 250c can redirect the energy E by a distance D, as measured by a length between the centers (e.g., the geometric centers) of the first and second regions 252c₁₋₂. The distance D can be between about 5 µm to about 5 mm, such as at least 5 µm, at least 10 µm, at least 20 µm, at least 30 µm, at least 40 µm, at least 50 µm, at least 100 µm, at least 500 µm, at least 1 mm, at least 2 mm, at least 3 mm, or any other suitable distance.

Although the system 200 is depicted as having two actuators 230 in FIGS. 2C-2E, in other embodiments the system 200 can include more or fewer actuators 230. In at least some embodiments, for example, the system 200 can include one, three, four, five, six, seven, eight, nine, ten, or any other suitable number of actuators 230. Although the system 200 is depicted as having four redirecting elements 250 in FIGS. 2A-2E, in other embodiments the system 200 can include more or fewer redirecting elements 250. In at least some embodiments, for example, the system 200 can include one, three, four, five, six, seven, eight, nine, ten, or any other suitable number of redirecting elements 250. In some embodiments, the number of redirecting elements corresponds to the number of actuation elements in the system.

Although the redirecting elements 250 are illustrated as having a parallelogram shape in FIGS. 2A-2E, in other embodiments the redirecting elements 250 can have other shapes. In at least some embodiments, for example, each of the redirecting elements 250 can have a circular, oval, curved, arcuate, curvilinear, triangular, square, rectangular, serpentine, zig-zag, rectilinear, pentagonal, hexagonal, or any other suitable shape.

As one skilled in the art will appreciate, any of the actuators and/or redirecting elements described above can be used as part of an adjustable shunting system, e.g., to control the flow of fluid therethrough. Moreover, certain features described with respect to one actuator and/or redirecting element can be added or combined with another actuator and/or redirecting element. Accordingly, the present technology is not limited to the actuators and redirecting elements expressly identified herein. For example, the energy redirecting elements could be utilized with the adjustable shunting systems and actuation assemblies described in U.S. Patent Application No. 17/175,332, U.S. Patent App. Publication No. 2020/0229982, and International Patent Application Nos. PCT/US20/55144, PCT/US20/55141, PCT/US21/14774, PCT/US21/18601, PCT/US21/023238, and PCT/US21/27742. Accordingly, although the redirecting elements are described in the context of specific adjustable shunting systems and actuation assemblies, redirecting elements in accordance with embodiments of the present technology can be used with any of the adjustable shunting systems, actuation assemblies, and/or actuators in the patent applications previously referenced. Likewise, although the redirecting elements are described in the context of adjustable shunting systems, the redirecting elements described herein can be used to transmit/convey energy to selectively actuate shape memory actuators coupled to other types of medical devices.

### Conclusion

The above detailed description of embodiments of the technology are not intended to be exhaustive or to limit the technology to the precise form disclosed above. Although specific embodiments of, and examples for, the technology are described above for illustrative purposes, various equivalent modifications are possible within the scope of the technology as those skilled in the relevant art will recognize. For example, any of the features of the intraocular shunts described herein may be combined with any of the features of the other intraocular shunts described herein and vice versa. Moreover, although steps are presented in a given order, altemative embodiments may perform steps in a different order. The various embodiments described herein may also be combined to provide further embodiments.

From the foregoing, it will be appreciated that specific embodiments of the technology have been described herein for purposes of illustration, but well-known structures and functions associated with intraocular shunts have not been shown or described in detail to avoid unnecessarily obscuring the description of the embodiments of the technology. Where the context permits, singular or plural terms may also include the plural or singular term, respectively.

Unless the context clearly requires otherwise, throughout the description and the examples, the words "comprise," "comprising," and the like are to be construed in an inclusive sense, as opposed to an exclusive or exhaustive sense; that is to say, in the sense of "including, but not limited to." As used herein, the terms "connected," "coupled," or any variant thereof, means any connection or coupling, either direct or indirect, between two or more elements; the coupling of connection between the elements can be physical, logical, or a combination thereof. Additionally, the words "herein," "above," "below," and words of similar import, when used in this application, shall refer to this application as a whole and not to any particular portions of this application. Where the context permits, words in the above Detailed Description using the singular or plural number may also include the plural or singular number respectively. As used herein, the phrase "and/or" as in "A and/or B" refers to A alone, B alone, and A and B. Additionally, the term "comprising" is used throughout to mean including at least the recited feature(s) such that any greater number of the same feature and/or additional types of other features are not precluded. It will also be appreciated that specific embodiments have been described herein for purposes of illustration, but that various modifications may be made without deviating from the technology.

## Claims

1. An actuation assembly for use with a shunting system for an eye, the actuation assembly comprising:
an actuator (230) including an actuation element (232); and
an energy redirecting element (250) having a first end portion and a second end portion spaced apart from the first end portion, the energy redirecting element (250) being configured to redirect optical or laser energy received at the first end portion toward the second end portion,
wherein the second end portion of the energy redirecting element (250) is at least partially aligned with the actuation element (232) such that optical or laser energy received at the first end portion of the energy redirecting element (250) is redirected to the actuation element (232).

2. The actuation assembly of claim 1, wherein the energy redirecting element (250) includes one or more transmission components (252) including at least one of the following: (i) a prism, (ii) a mirror, and/or (iii) a reflective surface, and wherein the one or more transmission components (252) are configured to redirect optical or laser energy received at the first end portion toward the second end portion.

3. The actuation assembly of claim 1, wherein the energy redirecting element (250) includes an optical fiber, and wherein the optical fiber includes a transmission component (252) and an exterior component (254) at least partially surrounding the transmission component (252).

4. The actuation assembly of claim 3, wherein the transmission component (252) has a refractive index of at least 1.34.

5. The actuation assembly of claim 3 or 4, wherein the transmission component (252) has a refractive index of at least 1.6.

6. The actuation assembly of any of claims 3 to 5, wherein the transmission component (252) is formed from an optical epoxy and/or polydimethylsiloxane.

7. The actuation assembly of any of claims 3 to 5, wherein the exterior component (254) is formed from a glass or a silicone elastomer.

8. The actuation assembly of any of claims 3 to 7, wherein the exterior component (254) has a second refractive index less than a first refractive index of the transmission component (252).

9. The actuation assembly of claim 8, wherein the second refractive index is up to 1.34.

10. The actuation assembly of any of claims 2 to 9, wherein the transmission component (252) is positioned within an interior of the energy redirecting element (250).

11. The actuation assembly of any of claims 1 to 10, wherein the energy redirecting element (250) is configured to redirect the optical or laser energy a distance of at least 5 µm between the first end portion and the second end portion.

12. The actuation assembly of any of claims 1 to **11,** wherein the actuation element (232) is configured to be positioned in a first body region and the energy redirecting element (250) is configured to be positioned in a second body region different than the first body region.

13. The actuation assembly of any of claims 1 to 12, wherein the energy redirecting element (250) is configured to redirect optical or laser energy to the second end portion when optical or laser energy is applied to the first end portion at an incidence angle of between 0 degrees and 70 degrees.

14. The actuation assembly of any of claims 1 to 13, wherein the actuation element (232) is a first actuation element and the energy redirecting element (250) is a first energy redirecting element, the actuation assembly further comprising:
a second actuation element; and
a second energy redirecting element having a third end portion and a fourth end portion, the second energy redirecting element being configured to redirect optical or laser energy received at the third end portion toward the fourth end portion,
wherein the fourth end portion of the energy redirecting element is aligned with the second actuation element such that optical or laser energy received at the third end portion of the second energy redirecting element is redirected to the second actuation element.

15. The actuation assembly of claim 1, wherein the energy redirecting element (250) comprises a transmission component (252) including an input region in the first end portion, an output region in the second end portion, and an intermediate region extending therebetween and optically coupling the input and output regions, wherein the output region is aligned with a target of the actuation element (232).

16. The actuation assembly of claim 15, wherein the transmission component (252) includes one or more prisms, mirrors, and/or reflective surfaces positioned in the interior of the energy redirecting element.

17. The actuation assembly of claim 16, wherein the one or more prisms, mirrors, and/or reflective surfaces are separated by a vacuum, air, water, and/or any other suitable fluid and/or material that permits optical energy to be propagated therethrough.

18. The actuation assembly of any preceding claim, wherein the energy redirecting element (250) is configured to redirect optical energy received at the first end portion toward the second end portion and wherein the optical energy is laser energy.

## Patentansprüche

1. Betätigungsanordnung zur Verwendung mit einem Bypass-System für ein Auge, wobei die Betätigungsanordnung Folgendes umfasst:
einen Aktuator (230), der ein Betätigungselement (232) einschließt; und
ein Energieumlenkelement (250), das einen ersten Endabschnitt und einen zweiten Endabschnitt, der von dem ersten Endabschnitt beabstandet ist, aufweist, wobei das Energieumlenkelement (250) dazu konfiguriert ist, optische oder Laserenergie, die an dem ersten Endabschnitt empfangen wird, in Richtung des zweiten Endabschnitts umzulenken,
wobei der zweite Endabschnitt des Energieumlenkelements (250) mindestens teilweise mit dem Betätigungselement (232) derart ausgerichtet ist, dass optische oder Laserenergie, die an dem ersten Endabschnitt des Energieumlenkelements (250) empfangen wird, zu dem Betätigungselement (232) umgelenkt wird.

2. Betätigungsanordnung nach Anspruch 1, wobei das Energieumlenkelement (250) eine oder mehrere Übertragungskomponenten (252) einschließt, die mindestens eines der Folgenden einschließen: (i) ein Prisma, (ii) einen Spiegel und/oder (iii) eine reflektierende Oberfläche, und wobei die eine oder die mehreren Übertragungskomponenten (252) dazu konfiguriert sind, optische oder Laserenergie, die an dem ersten Endabschnitt empfangen wird, in Richtung des zweiten Endabschnitts umzulenken.

3. Betätigungsanordnung nach Anspruch 1, wobei das Energieumlenkelement (250) eine optische Faser einschließt, und wobei die optische Faser eine Übertragungskomponente (252) und eine äußere Komponente (254), die die Übertragungskomponente (252) mindestens teilweise umgibt, einschließt.

4. Betätigungsanordnung nach Anspruch 3, wobei die Übertragungskomponente (252) einen Brechungsindex von mindestens 1,34 aufweist.

5. Betätigungsanordnung nach Anspruch 3 oder 4, wobei die Übertragungskomponente (252) einen Brechungsindex von mindestens 1,6 aufweist.

6. Betätigungsanordnung nach einem der Ansprüche 3 bis 5, wobei die Übertragungskomponente (252) aus einem optischen Epoxid und/oder Polydimethylsiloxan gebildet ist.

7. Betätigungsanordnung nach einem der Ansprüche 3 bis 5, wobei die äußere Komponente (254) aus einem optischen Glas oder einem Silikonelastomer gebildet ist.

8. Betätigungsanordnung nach einem der Ansprüche 3 bis 7, wobei die äußere Komponente (254) einen zweiten Brechungsindex aufweist, der geringer ist als der erste Brechungsindex der Übertragungskomponente (252).

9. Betätigungsanordnung nach Anspruch 8, wobei der zweite Brechungsindex bis zu 1,34 beträgt.

10. Betätigungsanordnung nach einem der Ansprüche 2 bis 9, wobei die Übertragungskomponente (252) innerhalb eines Inneren des Energieumlenkelements (250) positioniert ist.

11. Betätigungsanordnung nach einem der Ansprüche 1 bis 10, wobei das Energieumlenkelement (250) dazu konfiguriert ist, die optische oder Laserenergie über einen Abstand von mindestens 5 µm zwischen dem ersten Endabschnitt und dem zweiten Endabschnitt umzulenken.

12. Betätigungsanordnung nach einem der Ansprüche 1 bis 11, wobei das Betätigungselement (232) dazu konfiguriert ist, in einem ersten Körperbereich positioniert zu sein, und das Energieumlenkelement (250) dazu konfiguriert ist, in einem zweiten Körperbereich, der sich von dem ersten Körperbereich unterscheidet, positioniert zu sein.

13. Betätigungsanordnung nach einem der Ansprüche 1 bis 12, wobei das Energieumlenkelement (250) dazu konfiguriert ist, optische oder Laserenergie zu dem zweiten Endabschnitt umzulenken, wenn die optische oder Laserenergie auf den ersten Endabschnitt mit einem Einfallswinkel von zwischen 0 Grad und 70 Grad angewendet wird.

14. Betätigungsanordnung nach einem der Ansprüche 1 bis 13, wobei das Betätigungselement (232) ein erstes Betätigungselement ist und das Energieumlenkelement (250) ein erstes Energieumlenkelement ist, wobei die Betätigungsanordnung ferner Folgendes umfasst:
ein zweites Betätigungselement; und
ein zweites Energieumlenkelement, das einen dritten Endabschnitt und einen vierten Endabschnitt aufweist, wobei das zweite Energieumlenkelement dazu konfiguriert ist, optische oder Laserenergie, die an dem dritten Endabschnitt empfangen wird, in Richtung des vierten Endabschnitts umzulenken,
wobei der vierte Endabschnitt des Energieumlenkelements mit dem zweiten Betätigungselement derart ausgerichtet ist, dass optische oder Laserenergie, die an dem dritten Endabschnitt des zweiten Energieumlenkelements empfangen wird, zu dem zweiten Betätigungselement umgelenkt wird.

15. Betätigungsanordnung nach Anspruch 1, wobei das Energieumlenkelement (250) eine Übertragungskomponente (252) umfasst, die einen Eingangsbereich in den ersten Endabschnitt einschließt, und einen Ausgangsbereich in dem zweiten Endabschnitt sowie einen Zwischenbereich, der sich dazwischen erstreckt und den Eingangs- und den Ausgangsbereich optisch koppelt, wobei der Ausgangsbereich mit einem Ziel des Betätigungselements (232) ausgerichtet ist.

16. Betätigungsanordnung nach Anspruch 15, wobei die Übertragungskomponente (252) ein oder mehrere Prismen, Spiegel und/oder reflektierende Oberflächen einschließt, die in dem Inneren des Energieumlenkelements positioniert sind.

17. Betätigungsanordnung nach Anspruch 16, wobei das eine oder die mehreren Prismen, Spiegel und/oder reflektierenden Oberflächen durch ein Vakuum, Luft, Wasser und/oder jedes andere geeignete Fluid und/oder Material getrennt sind, das (die) es optischer Energie erlaubt, sich da hindurch auszubreiten.

18. Betätigungsanordnung nach einem vorstehenden Anspruch, wobei das Energieumlenkelement (250) dazu konfiguriert ist, optische Energie, die an dem ersten Endabschnitt empfangen wird, in Richtung des zweiten Endabschnitts umzulenken, und wobei die optische Energie Laserenergie ist.

## Revendications

1. Ensemble d'actionnement destiné à être utilisé avec un système de dérivation pour un œil, l'ensemble d'actionnement comprenant :
un actionneur (230) incluant un élément d'actionnement (232) ; et
un élément de redirection d'énergie (250) ayant une première partie d'extrémité et une deuxième partie d'extrémité espacée de la première partie d'extrémité, l'élément de redirection d'énergie (250) étant configuré pour rediriger de l'énergie optique ou laser reçue au niveau de la première partie d'extrémité vers la deuxième partie d'extrémité,
dans lequel la deuxième partie d'extrémité de l'élément de redirection d'énergie (250) est au moins partiellement alignée avec l'élément d'actionnement (232) de sorte que de l'énergie optique ou laser reçue au niveau de la première partie d'extrémité de l'élément de redirection d'énergie (250) est redirigée vers l'élément d'actionnement (232).

2. Ensemble d'actionnement selon la revendication 1, dans lequel l'élément de redirection d'énergie (250) inclut un ou plusieurs composants de transmission (252) incluant au moins l'un des éléments suivants : (i) un prisme, (ii) un miroir, et/ou (iii) une surface réfléchissante, et dans lequel les un ou plusieurs composants de transmission (252) sont configurés pour rediriger de l'énergie optique ou laser reçue au niveau de la première partie d'extrémité vers la deuxième partie d'extrémité.

3. Ensemble d'actionnement selon la revendication 1, dans lequel l'élément de redirection d'énergie (250) inclut une fibre optique, et dans lequel la fibre optique inclut un composant de transmission (252) et un composant extérieur (254) entourant au moins partiellement le composant de transmission (252).

4. Ensemble d'actionnement selon la revendication 3, dans lequel le composant de transmission (252) a un indice de réfraction d'au moins 1,34.

5. Ensemble d'actionnement selon la revendication 3 ou 4, dans lequel le composant de transmission (252) a un indice de réfraction d'au moins 1,6.

6. Ensemble d'actionnement selon l'une quelconque des revendications 3 à 5, dans lequel le composant de transmission (252) est formé à partir d'un époxy optique et/ou d'un polydiméthylsiloxane.

7. Ensemble d'actionnement selon l'une quelconque des revendications 3 à 5, dans lequel le composant extérieur (254) est formé à partir d'un verre ou d'un élastomère de silicone.

8. Ensemble d'actionnement selon l'une quelconque des revendications 3 à 7, dans lequel le composant extérieur (254) a un deuxième indice de réfraction inférieur à un premier indice de réfraction du composant de transmission (252).

9. Ensemble d'actionnement selon la revendication 8, dans lequel le deuxième indice de réfraction va jusqu'à 1,34.

10. Ensemble d'actionnement selon l'une quelconque des revendications 2 à 9, dans lequel le composant de transmission (252) est positionné au sein d'un intérieur de l'élément de redirection d'énergie (250).

11. Ensemble d'actionnement selon l'une quelconque des revendications 1 à 10, dans lequel l'élément de redirection d'énergie (250) est configuré pour rediriger l'énergie optique ou laser sur une distance d'au moins 5 µm entre la première partie d'extrémité et la deuxième partie d'extrémité.

12. Ensemble d'actionnement selon l'une quelconque des revendications 1 à 11, dans lequel l'élément d'actionnement (232) est configuré pour être positionné dans une première région corporelle et l'élément de redirection d'énergie (250) est configuré pour être positionné dans une deuxième région corporelle différente de la première région corporelle.

13. Ensemble d'actionnement selon l'une quelconque des revendications 1 à 12, dans lequel l'élément de redirection d'énergie (250) est configuré pour rediriger de l'énergie optique ou laser vers la deuxième partie d'extrémité lorsque de l'énergie optique ou laser est appliquée à la première partie d'extrémité à un angle d'incidence compris entre 0 degrés et 70 degrés.

14. Ensemble d'actionnement selon l'une quelconque des revendications 1 à 13, dans lequel l'élément d'actionnement (232) est un premier élément d'actionnement et l'élément de redirection d'énergie (250) est un premier élément de redirection d'énergie, l'ensemble d'actionnement comprenant en outre :
un deuxième élément d'actionnement ; et
un deuxième élément de redirection d'énergie ayant une troisième partie d'extrémité et une quatrième partie d'extrémité, le deuxième élément de redirection d'énergie étant configuré pour rediriger de l'énergie optique ou laser reçue au niveau de la troisième partie d'extrémité vers la quatrième partie d'extrémité,
dans lequel la quatrième partie d'extrémité de l'élément de redirection d'énergie est alignée avec le deuxième élément d'actionnement de sorte que de l'énergie optique ou laser reçue au niveau de la troisième partie d'extrémité du deuxième élément de redirection d'énergie est redirigée vers le deuxième élément d'actionnement.

15. Ensemble d'actionnement selon la revendication 1, dans lequel l'élément de redirection d'énergie (250) comprend un composant de transmission (252) incluant une région d'entrée dans la première partie d'extrémité, une région de sortie dans la deuxième partie d'extrémité, et une région intermédiaire s'étendant entre celles-ci et couplant optiquement les régions d'entrée et de sortie, dans lequel la région de sortie est alignée avec une cible de l'élément d'actionnement (232).

16. Ensemble d'actionnement selon la revendication 15, dans lequel le composant de transmission (252) inclut un ou plusieurs prismes, miroirs, et/ou surfaces réfléchissantes positionnés à l'intérieur de l'élément de redirection d'énergie.

17. Ensemble d'actionnement selon la revendication 16, dans lequel les un ou plusieurs prismes, miroirs, et/ou surfaces réfléchissantes sont séparés par un vide, de l'air, de l'eau, et/ou tout autre fluide et/ou matériau approprié qui permettent à de l'énergie optique de se propager à travers ceux-ci.

18. Ensemble d'actionnement selon l'une quelconque des revendications précédentes, dans lequel l'élément de redirection d'énergie (250) est configuré pour rediriger de l'énergie optique ou laser au niveau de la première partie d'extrémité vers la deuxième partie d'extrémité et dans lequel l'énergie optique est de l'énergie laser.
